# EUROPEAN PATENT APPLICATION

(11) **EP 4 467 557 A1**
(43) Date of publication of application: **27.11.2024**
(21) Application number: 23740110.4
(22) Date of filing: 17.01.2023
(51) Int. Cl.: C07H 19/16, C07H 1/06, A61P 31/18, A61K 31/7076

(54) **CRYSTAL OF 4'-SUBSTITUTED NUCLEOSIDE, PREPARATION METHOD THEREFOR, COMPOSITION CONTAINING SAME, AND USE THEREOF**

(30) Priority: 17.01.2022 CN 202210049924
(71) Applicant: Henan Genuine Biotech Co., Ltd., Henan 467036 (CN)
(72) Inventor: DU, Jinfa, Pingdingshan, Henan 467036 (CN)
(74) Representative: Bandpay & Greuter
(86) International application number: PCT/CN2023/072478
(87) International publication number: WO 2023/134769

(57) **Abstract**

The present invention relates to the field of medicinal chemistry, and provides a crystal of 4'-substituted nucleoside, a preparation method therefor, a composition containing the crystal, and a use thereof. The crystal of the 4'-substituted nucleoside is a type-III crystal of compound (3). The type-III crystal shows excellent physical properties, such as easy preparation, stable crystal form and the like, and is convenient for large-scale production and quality control of drugs. The type-III crystal is suitable as a crystal form of compound (3) in drug preparation.

## Description

### TECHNICAL FIELD

The present disclosure relates to the field of pharmaceutical chemistry, specifically to a crystal of 4'-substituted nucleoside, and a preparation method therefor, a composition thereof, and use thereof.

### BACKGROUND

AIDS has become a maj or threat to human health. Human immunodeficiency virus (HIV) is replicated through processes such as adsorption, invasion and uncoating, reverse transcription, synthesis, assembly, release and maturation of integrated viral RNA and proteins, etc. Each of these links could be used as a target for HIV-inhibiting drugs. After more than 30 years of research, forty HIV therapeutic drugs including drug combinations have been approved by the US FDA for clinical use. According to the mechanisms of action of these drugs, they can be mainly divided into several major categories such as nucleoside and non-nucleoside reverse transcriptase inhibitors, protease inhibitors, invasion inhibitors, and integrase inhibitors. These drugs are effective in inhibiting the HIV replication, but none of them could cure AIDS. Moreover, after a period of treatment with these drugs, the drug resistance of viruses develops constantly, rendering the existing drug therapies ineffective. Therefore, the combination of HIV inhibitors with different mechanisms of action has become a standard strategy for treatment of AIDS. Nevertheless, even if drug combinations are used to treat AIDS, the new drug resistance continues to emerge. Thus, there still remains a need to continue to search for AIDS therapeutic drugs with new mechanisms of action. Additionally, to date, all AIDS therapeutic drugs have to be taken at least once daily and long-term medication has become a burden for patients. Therefore, it is necessary to develop long-acting AIDS therapeutic drugs.

2'-Deoxynucleosides are the main first-in-class HIV inhibitors used in clinical practice, and they exert their drug effects by inhibiting the reverse transcriptase. At present, the nucleoside HIV inhibitors for clinical use are all 3'-deoxynucleosides. Owing to the absence of 3'-OH, the drug terminates the HIV DNA elongation after embedded in the HIV DNA, thereby becoming a terminator of HIV DNA synthesis and thus achieving the effect of inhibiting the HIV replication. In recent years, nucleosides with 3'-OH, such as compound (I) (C.A. Stoddart et al Antimicrob. Agents Chemother. 2015, 59, 4190) and compound (II) (Q .Wang et al Eur. J. Med. Chem. 2011, 46, 4178) have been successively reported to have significant inhibitory activities against HIV Their structures are characterized by the presence of a large substituent at the 4'-position, such as the ethynyl group in compound (I) and the azide group in compound (2). As a result of the introduction of these large substituents, the nucleoside phosphates are bound into the viral DNA, and then the elongation of viral DNA becomes slow or terminates due to the steric hindrance effect, thereby fulfilling the purpose of inhibiting the HIV replication.

Among adenine nucleosides, the introduction of 2-fluorine increases the HIV inhibitory activity (EC₅₀ = 11 nM for EdA) of 4'-ethynyl-2'-deoxyadenosine (EC₅₀ = 0.05 nM for Compound 1, EFdA) by 2200 times (E. Michailidis et al J. Biol. Chem. 2009, 284,35681).

The U.S. patent (J. Chang US8835615, 2014) discloses the chemical structure of compound (3) (2R,3R,4S,5R)-5-(6-amino-2-fluoro-9H-purin-9-yl)-2-ethynyl-4-fluoro-2-(hydroxymethyl)tetrahydrofuran-3-ol. Subsequently, the Chinese patent (Junbiao CHANG, CN109893536 B, 2021) discloses the anti-HIV activity (EC₅₀ = 0.9 nM) and the safety (without significant cytotoxicity within the test dose range, CC₅₀>8000 nM) of compound **(3),** and also discloses the crystal form A of compound (3). The DSC and TGA studies have discovered that the molecular weight of the compound is 311.24, and the weight loss of the crystal form A is 9.0% at 110°C, indicating that crystal form A contains a solvated crystal of one molecule of methanol (theoretical methanol content: 9.33%), and methanol will be lost upon heating.

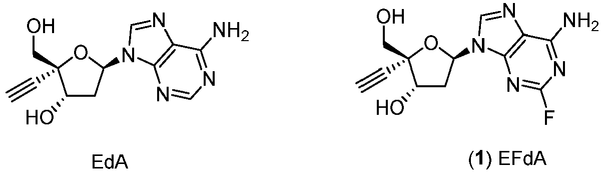

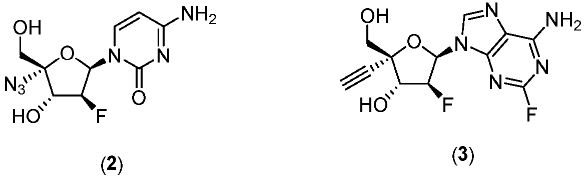

### Structures of Some Nucleoside Compounds under Research

### SUMMARY

The present inventors have conducted intensive studies on the crystal form of the compound (3) and found that compound (3) forms numerous crystal forms under different conditions. However, a majority of the crystal forms are unstable. The present inventors have unexpectedly found the type-III crystal exhibits very excellent physical properties, such as ease of preparation and stable crystal form, which is convenient for mass production and quality control of drugs and is very suitable as a crystal form of compound (3) in the preparation of a drug.

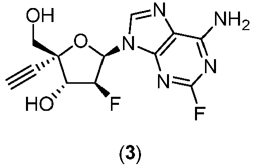

In view of the foregoing, the present disclosure provides a type-III crystal of compound (3). The type-III crystal shows characteristic peaks at diffraction angles of 20 values = 8.83°, 16.08°, 16.53°, 17.67°, 18.19°, 25.52°, 26.20°, and 27.05° in an X-ray powder diffraction pattern obtained by using Cu-Kα radiation, wherein the 20 values are allowed to vary within an error range.

The data on the diffraction peaks of the type-III crystal of compound (3) are as shown in Table 1 below.

**Table 1. XRPD Data on Type-III Crystal**

| Peak Pos. [°2θ] | Rel. Int. [%] |
|---|---|
| 8.83 | 100.00 |
| 16.08 | 39.90 |
| 16.53 | 51.80 |
| 17.67 | 98.71 |
| 18.19 | 25.29 |
| 25.52 | 38.72 |
| 26.20 | 44.53 |
| 27.05 | 67.31 |

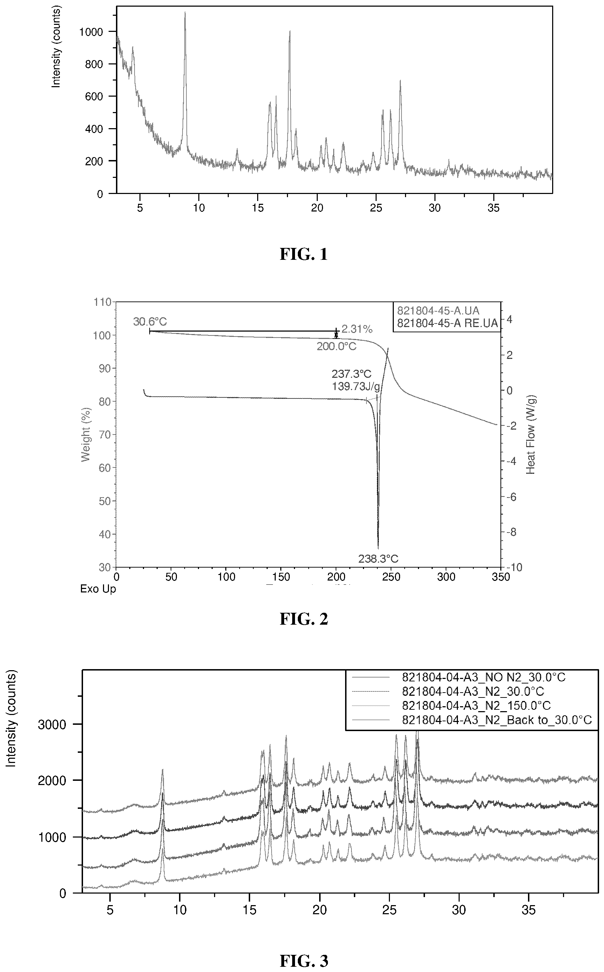

The XRPD pattern of the type-III crystal is as shown in FIG. 1.

The TGA/DSC diagram for the type-III crystal is as shown in FIG. 2.

The variable-temperature XRPD comparison diagram for the type-III crystal is as shown in FIG. 3.

In another aspect, the present disclosure provides a method for preparing a type-III crystal of compound (3), comprising: dissolving the compound (3) into a solution of 1,4-dioxane/DCM (1-5/10), adding petroleum ether or lower paraffin hydrocarbons, which may include but not limited to pentane, hexane, octane, nonane, or decane or the like, to the solution, leaving or stirring for 1 to 24 h, preferably 3 to 10 h, more preferably 4 h to precipitate a crystal, and filtering; subjecting the resulting crystal to vacuum treatment at 25°C to 120°C, preferably 40°C to 60°C for 1 to 24 h, preferably 3 to 5 h to afford the type-III crystal.

The present disclosure further provides another method for preparing a type-III crystal of compound (3), comprising: stirring the compound (3) in ethanol at room temperature for 2 to 72 h, preferably 20 to 60 h, more preferably 48 h, filtering, vacuum drying at room temperature to afford a crystal, heating the resulting crystal to 120°C to 150°C, preferably 140°C to 145°C, more preferably 141°C under nitrogen protection to afford the type-III crystal of compound (3).

The present disclosure further provides another method for preparing a type-III crystal of compound (3), comprising: dissolving the compound (3) into THF or methyl THF, adding petroleum ether or lower paraffin hydrocarbons, which may include but not limited to, e.g., pentane, hexane, octane, nonane, and decane, to the resulting solution, and adding an appropriate amount of type III as a seed crystal to obtain a white suspension, stirring or leaving the resulting suspension at 40°C to 70°C, preferably 50°C for 1 to 4 h, preferably 60 to 70 min, and then slowly cooling to room temperature, and stirring or standing still for appropriate time; filtering the resulting suspension under reduced pressure at room temperature, washing with n-heptane, vacuum drying at 40°C to 80°C, preferably 50°C to afford the type-III crystal.

In another aspect, the present disclosure further provides a pharmaceutical composition, comprising the type-III crystal of compound (3) described above and an optional pharmaceutical excipient.

In another aspect, the present disclosure further provides use of a type-III crystal of compound (3), or the above pharmaceutical composition comprising the type-III crystal of the compound (3) and an optional pharmaceutical excipient in the preparation of a pharmaceutical or pharmaceutical formulation for resisting HIV or treating AIDS.

The present disclosure further provides a method for resisting HIV or treating AIDS, comprising administering, to a subject, an anti-HIV or therapeutically effective dose of a type-III crystal of compound (3) or the above pharmaceutical composition comprising the type-III crystal of the compound (3) and an optional pharmaceutical excipient.

### Advantageous Effects of the Invention

The present inventors have prepared a myriad of crystal forms of compound (3), most of which are unstable solid forms. The type-III crystal of compound (3) exhibits excellent physical properties, such as ease of preparation and stable crystal form, which facilitates mass production and quality control of drugs and suitable as the crystal form of compound (3) in the drug preparation.

### BRIEF DESCRIPTION OF THE DRAWINGS

FIG. 1 shows the XRPD pattern of the type-III crystal.
FIG. 2 shows the TGA/DSC diagram for the type-III crystal.
FIG. 3 shows the variable-temperature XRPD comparison diagram for the type-III crystal.
FIG. 4 shows the XRPD pattern of the type-I crystal.
FIG. 5 shows the TGA/DSC diagram for the type-I crystal.
FIG. 6 shows the XRPD comparison diagram before and after heating the type-I crystal to 55°C.
FIG. 7 shows the XRPD pattern of the type-I crystal when heated to 100°C.
FIG. 8 shows the XRPD pattern of the type-II crystal.
FIG. 9 shows the XRPD pattern of the type-IV crystal.
FIG. 10 shows the XRPD pattern of the type-V crystal.
FIG. 11 shows the XRPD pattern of the type-VI crystal.
FIG. 12 shows the XRPD pattern of the type-VII crystal.
FIG. 13 shows the XRPD pattern of the type-VIII crystal.
FIG. 14 shows the XRPD pattern of the type-IX crystal.
FIG. 15 shows the XRPD pattern of the type-X crystal.
FIG. 16 shows the XRPD pattern of the type-XI crystal.
FIG. 17 shows the XRPD pattern of the type-XII crystal.
FIG. 18 shows the XRPD pattern of the type-XIII crystal.
FIG. 19 shows the XRPD comparison diagrams before and after coating and drying the type-XIII crystal.
FIG. 20 shows the XRPD pattern of the type-XIV crystal.
FIG. 21 shows the XRPD pattern of the type-XV crystal.
FIG. 22 shows the XRPD pattern of the type-XVI crystal.
FIG. 23 shows the DSC-TGA diagram for the type-XVI crystal.
FIG. 24 shows the three-dimensional view of single-crystal X-ray diffraction molecules of the type-XVI crystal.
FIG. 25 shows the XRPD pattern of the type-XVII crystal.
FIG. 26 shows the DSC-TGA diagram for the type-XVII crystal.

### DETAILED DESCRIPTION

The present disclosure will be further elaborated below with reference to specific experiments. It should be appreciated that these experiments are merely intended to illustrate the present disclosure and are not intended to limit the scope of protection for the present disclosure. Experimental methods for which no specific conditions are indicated in the following experiments are generally performed according to conventional conditions or the conditions recommended by the manufacturers. Unless otherwise defined, all professional and scientific terms used herein have the same meanings as those familiar to the technicians in the art. In addition, any methods and materials similar or equivalent to those described herein may be applied to the methods of the present disclosure. The preferred implementation methods and materials as shown herein are for illustrative purposes only.

The starting materials used in the experiments of the present disclosure are known and commercially available, or may be synthesized by the methods known in the art.

### Experiment 1

### Preparation of Type-III Crystal

The compound (3) as a solid (150 mg) was dissolved in a 1,4-dioxane/DCM solution. To the solution, n-heptane was added dropwise, stirred for 4 h, and filtered to obtain a crystal. The resulting crystal was vacuum dried at room temperature for half an hour. The XRPD results showed that the type-III crystal was obtained.

### Experiment 2

### Preparation of Type-III Crystal

The compound (3) as a solid was suspended with stirring in ethanol at room temperature for 48 h, filtered, and vacuum dried at room temperature to obtain a crystal. The resulting crystal was heated to 141°C under nitrogen protection. The XRPD results showed that the type-III crystal of compound (3) was obtained.

### Experiment 3

### Scale Preparation of Type-III Crystal

Compound 3 (1575.3 mg) was weighed and put in a 100-mL reactor, and THF (35 mL) was added to the reactor with mechanical stirring (at a rotational speed of about 320 rpm), and dissolved at 50°C to obtain a clear solution.

To the reactor, n-heptane (48 mL) was added dropwise, during which an appropriate amount of the type-III crystal was added in batches as a seed crystal to obtain a white suspension. The resulting suspension was balanced at 50°C for 70 min, slowly cooled to 25°C at a rate of 0.5°C/min, and then stirred for about 3 days.

The resulting suspension was subj ected to suction filtration under reduced pressure, and washed with n-heptane (3 mL). After suction filtration, the resultant was transferred to 50°C and vacuum dried for about 5 h to obtain 1.2 g of white solid. The white solid was characterized by XRPD. The results showed that the crystal form (III) was obtained.

The data on the diffraction peaks of the type-III crystal of compound (3) were as shown in Table 1 above.

The XRPD pattern of the type-III crystal was as shown in FIG. 1.

The TGA/DSC diagram for the type-III crystal was as shown in FIG. 2.

The variable-temperature XRPD comparison diagram for the type-III crystal was as shown in FIG. 3.

TGA/DSC showed that the type-III crystal was heated to 200°C with a weight loss of 1.2%. The analysis showed that the weight loss was caused by a small amount of residual solvent or water from the sample surface. One endothermic signal was observed at 237.9°C (initial temperature). The HPLC results showed that the purity of the sample was 99.4 area%. The yield was 68.3%. TGA(110°C) showed that the sample was stable. The variable-temperature XRPD results were as shown in FIG. 3. Under nitrogen protection, after the type-III crystal was heater to 150°C, the crystal form did not change. After analysis, the type-III crystal was determined as an anhydrous stable crystal form.

### Experiment 4

### Type-I Crystal

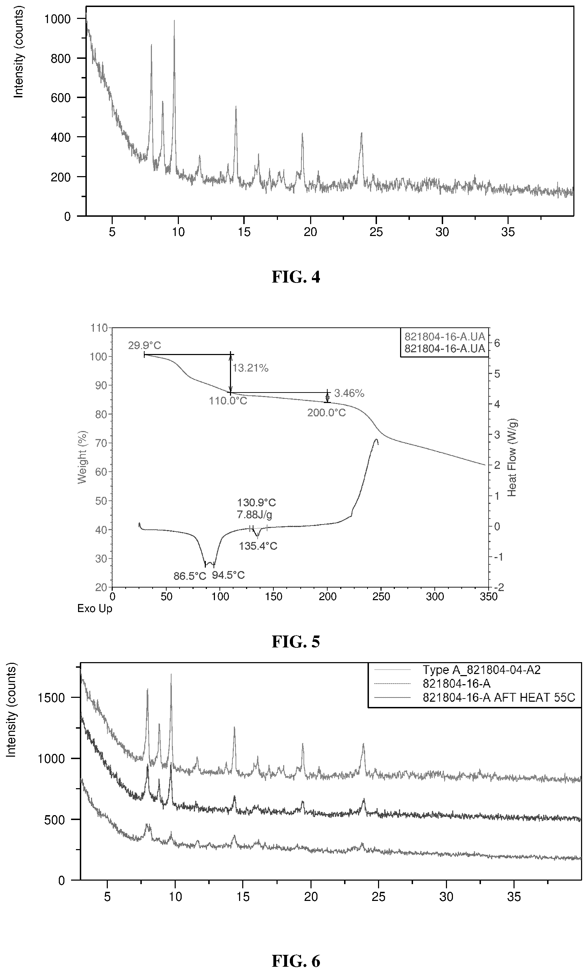

The compound (3) as a solid was dissolved in 2-MeTHF, and n-heptane was added dropwise at room temperature, stirred for 4 h to precipitate a crystal. The crystal was filtered, and vacuum dried to afford the type-I crystal of the compound (3). The XRPD pattern was as shown in FIG. 4.

The type-I crystal of the compound (3) showed characteristic peaks at diffraction angles of 2θ values = 7.94°, 8.79°, 9.68°, 11.56°, 13.74°, 14.37°, 15.98°, 17.78°, 19.39°, and 23.85° in an X-ray powder diffraction pattern obtained by using Cu-Kα radiation.

**Table 2. XRPD Data of Type-I Crystal**

| Peak Pos. [°2θ] | Rel. Int. [%] |
|---|---|
| 7.94 | 79.23 |
| 8.79 | 44.33 |
| 9.68 | 100.00 |
| 11.56 | 11.92 |
| 13.74 | 11.45 |
| 14.37 | 51.34 |
| 15.98 | 12.22 |
| 17.78 | 4.68 |
| 19.39 | 33.41 |
| 23.85 | 35.60 |

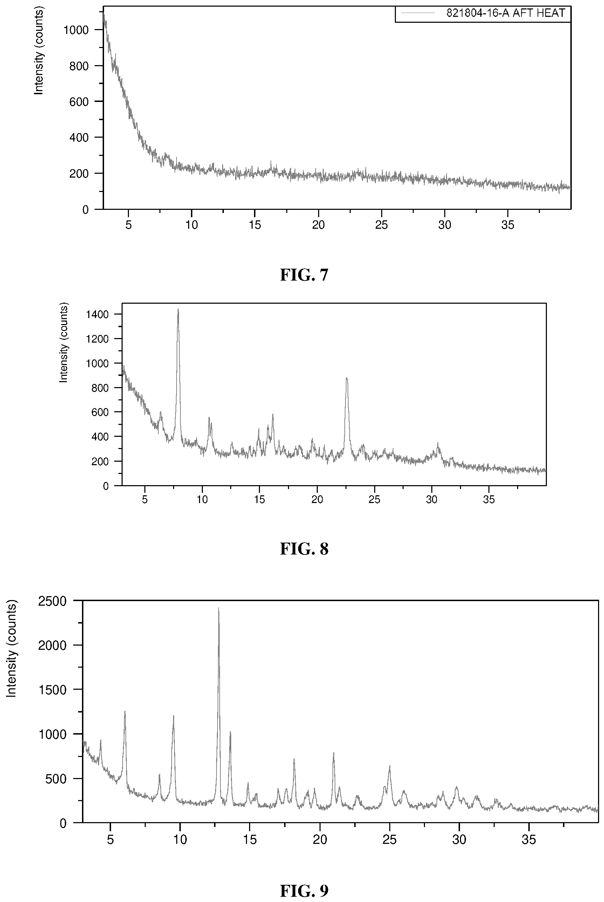

The TGA/DSC diagram for the type-I crystal of compound (3) was as shown in FIG. 5. The crystal was heated to 110°C with a weight loss of 13.2%, and further heated to 200°C with a weight loss of 3.5%. There were three endothermic signals in the range of 80°C to 140°C. Under nitrogen protection, the type-I crystal was heated to 55°C and then cooled to room temperature. The sample was removed and exposed to the air to test XRPD. The results were as shown in FIG. 6, where the upper curve was the original picture of the crystal form I, the middle curve was the XRPD pattern before heating, and the lower curve was the XRPD pattern after heating to 55°C, showing that the diffraction peak intensity of the sample was somewhat reduced. The NMR results showed that the molar ratio of 2-MeTHF to API was 0.25:1.00 (6.5 wt%) after heating, and 2-MeTHF was reduced by about 3.5 wt% as compared to that before heating. After the type-I crystal was heated to 100°C under the same conditions of the nitrogen protection, the XRPD results were as shown in FIG. 7, indicating that the crystal had been transformed into an amorphous crystal. The NMR results showed that the molar ratio of 2-MeTHF to API was 0.09:1.00 (2.4 wt%) after heating, and 2-MeTHF was reduced by about 7.6 wt% as compared to that before heating.

### Experiment 5

### Type-II Crystal

The compound (3) as a solid was dissolved in a 1,4-dioxane/DCM (1-5/10) solution, and n-heptane was added dropwise to the solution, stirred for 4 h to precipitate a crystal. The crystal was filtered to prepare the type-II crystal of the compound (3). The XRPD pattern was as shown in FIG. 8.

The type-II crystal showed characteristic peaks at diffraction angles of 20 values = 6.39°, 7.84°, 10.58°, 12.57°, 14.88°, 15.71°, 16.12°, 19.6°, 22.57°, 23.91°, and 30.62° in an X-ray powder diffraction pattern obtained by using Cu-Kα radiation.

**Table 3. XRPD Data of Type-II Crystal**

| Peak Pos. [°2θ] | Rel. Int. [%] |
|---|---|
| 6.39 | 12.49 |
| 7.84 | 100.00 |
| 10.58 | 26.61 |
| 12.57 | 8.65 |
| 14.88 | 15.36 |
| 15.71 | 22.47 |
| 16.12 | 31.30 |
| 19.60 | 8.47 |
| 22.57 | 62.65 |
| 23.91 | 6.93 |
| 30.62 | 10.91 |

After the type-II crystal was vacuum dried at room temperature for half an hour, the crystal form changed and transformed into a type-III crystal. As the type-II crystal was unstable, the crystal had been transformed after drying for a short period of time, and therefore the type-II crystal was not further studied.

### Experiment 6

### Type-IV Crystal

The compound (3) as a solid was suspended with stirring in EtOH at room temperature for 48 h, and filtered to afford the type-IV crystal. The XRPD pattern was as shown in FIG. 9.

The type-IV crystal showed characteristic peaks at diffraction angles of 2θ values = 4.28°, 6.03°, 8.50°, 9.53°, 12.76°, 13.58°, 14.84°, 15.45°, 17.02°, 17.61°, 18.17°, 19.12°, 19.60°, 20.97°, 21.38°, 22.66°, 24.99°, 25.99°, 28.47°, 28.82°, 29.80°, 31.24°, 32.66°, and 36.78° in an X-ray powder diffraction pattern obtained by using Cu-Kα radiation.

**Table 4. XRPD Data of Type-IV Crystal**

| Peak Pos. [°2θ] | Rel. Int. [%] |
|---|---|
| 4.28 | 11.98 |
| 6.03 | 38.66 |
| 8.50 | 11.78 |
| 9.53 | 41.18 |
| 12.76 | 100.00 |
| 13.58 | 37.85 |
| 14.84 | 10.63 |
| 15.45 | 6.39 |
| 17.02 | 8.33 |
| 17.61 | 9.35 |
| 18.17 | 24.53 |
| 19.12 | 7.80 |
| 19.60 | 9.64 |
| 20.97 | 28.26 |
| 21.38 | 11.19 |
| 22.66 | 604 |
| 24.99 | 21.87 |
| 25.99 | 904 |
| 28.47 | 6.38 |
| 28.82 | 8.04 |
| 29.80 | 11.07 |
| 31.24 | 5.98 |
| 32.66 | 4.22 |
| 36.78 | 1.46 |

After the type-IV crystal was vacuum dried at room temperature for 15 min, decreased relative intensity of the diffraction peaks could be observed. After the dried crystal was left closed at room temperature for about 5 days, increased relative intensity of the diffraction peaks and increased number of diffraction peaks could be observed. The analysis showed that the type-IV crystal might undergo solvent or water removal during vacuum drying, and adsorb the moisture in the air upon contact with air, thereby increasing the degree of crystallinity.

TGA showed that the type-IV crystal was unstable at 120°C with the weight loss of 10.6%, which was a monohydrate crystal form. Under the conditions of nitrogen protection, the type-IV crystal was heated to 141°C and then tested for XRPD. The results showed that the type-IV crystal was transformed into a stable type-III crystal at 141°C.

### Experiment 7

### Type-V Crystal

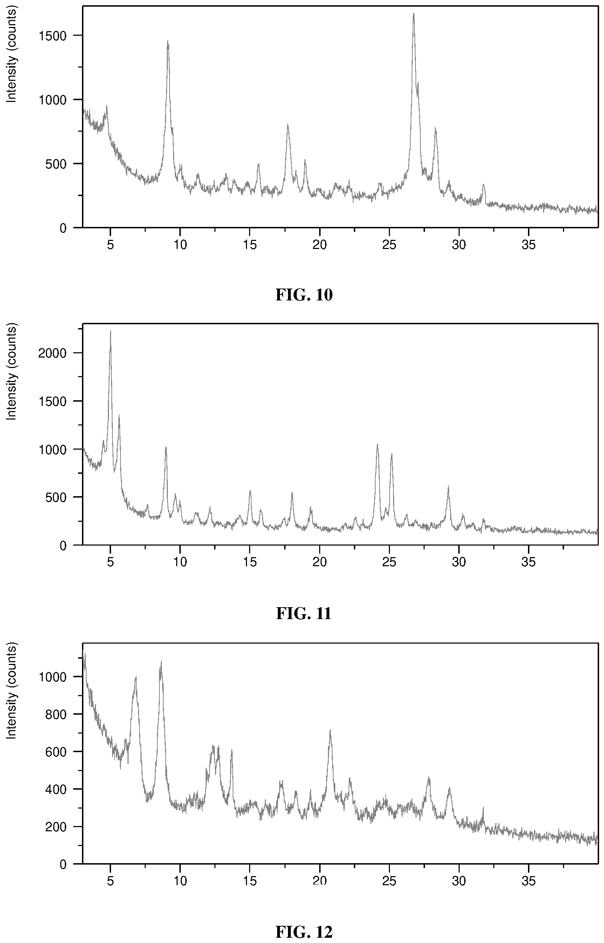

The compound (3) as a solid was suspended with stirring in EtOAc at room temperature for 48 h, filtered, and vacuum dried at room temperature for 15 min to afford the type-V crystal. The XRPD pattern was as shown in FIG. 10.

The type-V crystal showed characteristic peaks at diffraction angles of 20 values = 4.73°, 9.12°, 11.27°, 13.22°, 15.58°, 17.69°, 18.95°, 21.13°, 22.10°, 24.29°, 26.72°, 27.09°, 28.34°, 29.25°, and 31.76° in an X-ray powder diffraction pattern obtained by using Cu-Kα radiation.

**Table 5. XRPD Data of Type-V Crystal**

| Peak Pos. [°2θ] | Rel. Int. [%] |
|---|---|
| 4.73 | 18.22 |
| 9.12 | 73.14 |
| 11.27 | 6.49 |
| 13.22 | 6.45 |
| 15.58 | 14.84 |
| 17.69 | 36.96 |
| 18.95 | 17.63 |
| 21.13 | 6.37 |
| 22.10 | 6.14 |
| 24.29 | 8.50 |
| 26.72 | 100.00 |
| 27.09 | 54.66 |
| 28.34 | 37.44 |
| 29.25 | 10.32 |
| 31.76 | 10.93 |

The TGA/DSC test results showed that the type-V crystal was heated to 140°C with the weight loss of 15.4%, and further heated to 240°C with the weight loss of 2.8%. Multiple thermal signals could be observed in the range of 77°C to 203°C. The NMR results showed that the molar ratio of EtOAc to API was 0.40:1.00 (10.2 wt%).

The type-V crystal was subjected to the variable-temperature XRPD test. A significant decrease in degree of crystallinity was observed after the sample was purged with nitrogen for about 20 min. The sample was substantially transformed into an amorphous crystal after heating to 90°C and 130°C under the nitrogen protection, and after the crystal was further heated to 154°C, it was transformed into another crystal. Analysis of the NMR and TGA results showed that the type-V crystal was a solvate.

### Experiment 8

### Type-VI Crystal

The compound (3) as a solid was suspended with stirring in MIBK (methyl isobutyl ketone) at room temperature for 48 h, filtered, and vacuum dried at room temperature for 15 min to afford the type-VI crystal. The XRPD was as shown in FIG. 11.

The type-VI crystal showed characteristic peaks at diffraction angles of 20 values = 5.01°, 5.62°, 7.62°, 8.98°, 9.67°, 9.99°, 11.11°, 12.12°, 14.22°, 15.02°, 15.79°, 18.01°, 19.37°, 22.55°, 24.18°, 25.15°, 26.22°, 29.21°, 30.29°, 31.74°, and 34.00° in an X-ray powder diffraction pattern obtained by using Cu-Kα radiation.

**Table 6. XRPD Data of Type-VI Crystal**

| Peak Pos. [°2θ] | Rel. Int. [%] |
|---|---|
| 5.01 | 100.00 |
| 5.61 | 52.70 |
| 7.62 | 6.47 |
| 8.98 | 47.48 |
| 9.67 | 15.82 |
| 9.99 | 11.86 |
| 11.11 | 6.94 |
| 12.12 | 950 |
| 14.22 | 5.81 |
| 15.02 | 22.06 |
| 15.79 | 10.91 |
| 18.01 | 21.26 |
| 19.37 | 11.80 |
| 22.55 | 7.32 |
| 24.18 | 52.18 |
| 25.15 | 48.50 |
| 26.22 | 8.15 |
| 29.21 | 27.36 |
| 30.29 | 8.21 |
| 31.74 | 6.34 |
| 34.00 | 1.75 |

The TGA/DSC results showed that the type-VI crystal was heated to 70°C with the weight loss of 4.9%, and further heated to 130°C with the weight loss of 5.9%. Multiple thermal signals could be observed in the range of 98°C to 210°C.

The type-VI crystal was heated to 160°C under the nitrogen protection. The XRPD results showed that the crystal form was transformed.

### Experiment 9

### Type-VII Crystal

At room temperature, the compound (3) as a solid was suspended with stirring for 48 h in an IPA/toluene (v/v, 1: 1) mixed solvent, filtered, and vacuum dried at room temperature for 15 min to afford the type-VII crystal. The XRPD results were as shown in FIG. 12.

The type-VII crystal showed characteristic peaks at diffraction angles of 2θ values = 6.80°, 8.59°, 12.36°, 12.77°, 13.68°, 17.24°, 18.30, 19.32°, 20.77°, 22.20°, 24.47°, 27.81°, 29.32°, and 31.67° in an X-ray powder diffraction pattern obtained by using Cu-Kα radiation.

**Table 7. XRPD Data of Type-VII Crystal**

| Peak Pos. [°2θ] | Rel. Int. [%] |
|---|---|
| 6.80 | 76.64 |
| 8.59 | 100.00 |
| 12.36 | 45.49 |
| 12.77 | 38.03 |
| 13.68 | 47.03 |
| 17.24 | 22.07 |
| 18.30 | 17.43 |
| 19.32 | 13.81 |
| 20.77 | 63.08 |
| 22.20 | 25.93 |
| 24.47 | 6.55 |
| 27.81 | 27.02 |
| 29.32 | 23.87 |
| 31.67 | 8.90 |

The TGA/DSC test results showed that the type-VII crystal was heated to 130°C with the weight loss of 11.2%, and multiple thermal signals could be observed within the range of 88°C to 215°C. Under the nitrogen protection, the type-VII crystal was heated to 130°C and then cooled to room temperature. The sample was removed and exposed to the air to test XRPD. The results showed that the crystal form was transformed.

### Experiment 10

### Type-VIII Crystal

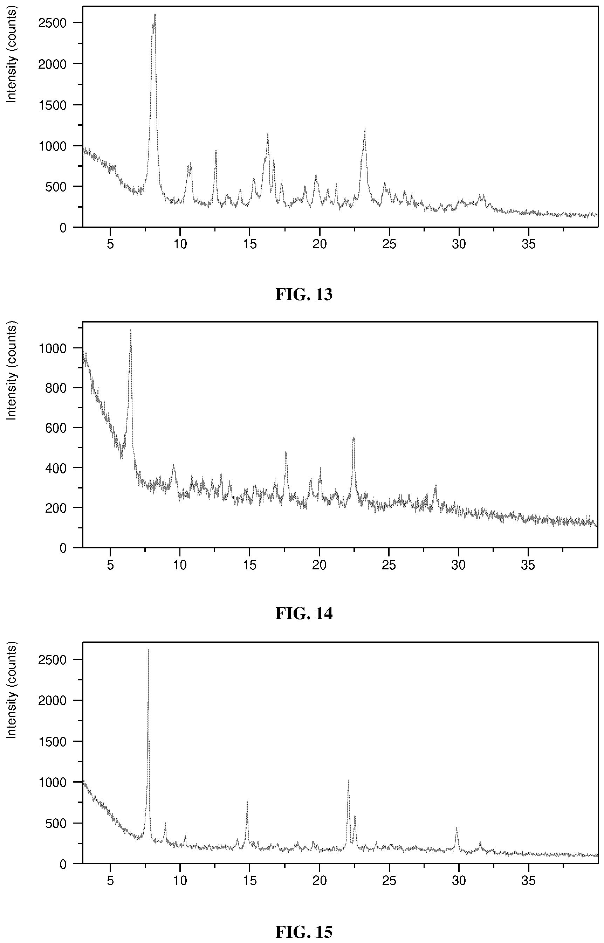

The compound (3) as a solid was suspended with stirring in a THF/n-heptane (v/v, 1: 1) mixed solvent at 50°C for 48 h, filtered, and vacuum dried at room temperature for 15 min to afford the type-VIII crystal. The XRPD pattern was as shown in FIG. 13.

The **type-VIII crystal** showed characteristic peaks at diffraction angles of 20 values = 5.24°, 7.98°, 8.20°, 10.51°, 10.77°, 12.55°13.40°, 14.27°, 15.28°, 16.29°, 16.69°, 17.25°, 18.94°, 19.72°, 21.19°, 23.23°, 24.63°, 26.09°, 26.60°, 30.05°, and 31.77° in an X-ray powder diffraction pattern obtained by using Cu-Kα radiation.

**Table 8. XRPD Diffraction Peak Data on Type-VIII Crystal**

| Peak Pos. [°2θ] | Rel. Int. [%] |
|---|---|
| 5.24 | 4.06 |
| 7.98 | 95.96 |
| 8.20 | 100.00 |
| 10.51 | 16.80 |
| 10.77 | 19.75 |
| 12.55 | 2904 |
| 13.40 | 4.75 |
| 14.27 | 8.18 |
| 15.28 | 15.07 |
| 16.29 | 38.51 |
| 16.69 | 25.75 |
| 17.25 | 13.01 |
| 18.94 | 10.57 |
| 19.72 | 17.57 |
| 21.19 | 11.64 |
| 23.23 | 43.54 |
| 24.63 | 13.72 |
| 26.09 | 908 |
| 26.60 | 8.13 |
| 30.05 | 3.73 |
| 31.77 | 7.96 |

The TGA/DSC results showed that the type-VIII crystal was heated to 160°C with the weight loss of 10.6%, and multiple thermal signals could be observed within the range of 120°C to 220°C. Under the nitrogen protection, the type-VIII crystal was heated to 160°C and then cooled to room temperature. The sample was removed and exposed to the air to test XRPD. The results showed that the crystal form had been transformed.

### Experiment 11

### Type-IX Crystal

At room temperature, the compound (3) was dissolved in a THF/CHCl₃ mixed solvent and volatilized at room temperature to afford the type-IX crystal. The XRPD pattern was as shown in FIG. 14.

The type-IX crystal showed characteristic peaks at diffraction angles of 20 values = 6.47°, 9.57°, 13.56°, 16.84°, 17.61°, 19.36°, 20.06°, 22.45°, and 28.32° in an X-ray powder diffraction pattern obtained by using Cu-Kα radiation.

**Table 9. XRPD Data on Type-IX Crystal**

| Peak Pos. [°2θ] | Rel. Int. [%] |
|---|---|
| 6.47 | 100.00 |
| 9.57 | 17.93 |
| 13.56 | 8.20 |
| 16.84 | 9.22 |
| 17.61 | 35.46 |
| 19.36 | 15.63 |
| 2006 | 21.60 |
| 22.45 | 49.26 |
| 28.32 | 13.96 |

The TGA/DSC results showed that the type-IX crystal was heated to 160°C with the weight loss of 20.3%, and multiple thermal signals could be observed within the range of 130°C to 215°C. After the type-IX crystal was left closed at room temperature for about 3 days, a reduced degree of crystallinity could be observed. The crystal was heated to 95°C under nitrogen protection and then cooled to room temperature. The sample was removed and exposed to the air to test XRPD. The results showed that the degree of crystallinity was further decreased. The crystal was heated to 160°C under the same conditions of nitrogen protection and then cooled to room temperature. The sample was removed and exposed to the air to test XRPD. The results showed that its crystal form had been transformed. The analysis of the NMR and TGA results showed that after heating, the change in crystal form was caused by solvent removal, and the IX crystal was a solvate.

### Experiment 12

### Type-X Crystal

The compound (3) as a solid was suspended with stirring in IPAc (isopropyl acetate) at 50°C for 48 h, and filtered to afford the type-X crystal. The XRPD pattern was as shown in FIG. 15.

The type-X crystal showed characteristic peaks at diffraction angles of 20 values = 7.72°, 8.91°, 14.10°, 14.79°, 16.95°, 18.39°, 19.53°, 22.06°, 22.53°, 29.81°, and 31.51° in an X-ray powder diffraction pattern obtained by using Cu-Kα radiation.

**Table 10. XRPD Data on Type-X Crystal**

| Peak Pos. [°2θ] | Rel. Int. [%] |
|---|---|
| 7.72 | 100.00 |
| 8.91 | 8.59 |
| 14.10 | 4.77 |
| 14.79 | 24.51 |
| 16.95 | 2.57 |
| 18.39 | 2.79 |
| 19.53 | 4.00 |
| 22.06 | 35.63 |
| 22.53 | 17.81 |
| 29.81 | 12.77 |
| 31.51 | 4.98 |

The TGA/DSC results showed that the type-X crystal was heated to 130°C with the weight loss of 7.9%, and was further heated to 160°C with the weight loss of 4.5%. Multiple thermal signals could be observed within the range of 126°C to 210°C.

The type-X crystal was subjected to the variable-temperature XRPD test. The results showed that the position of the diffraction peak shifted after the type-X crystal was heated to 120°C while purging with nitrogen; the diffraction peak intensity was significantly reduced after the type-X crystal was further heated to 130°C; and the crystal form was transformed after the type-X crystal was further heated to 145°C.

### Experiment 13

### Type-XI Crystal

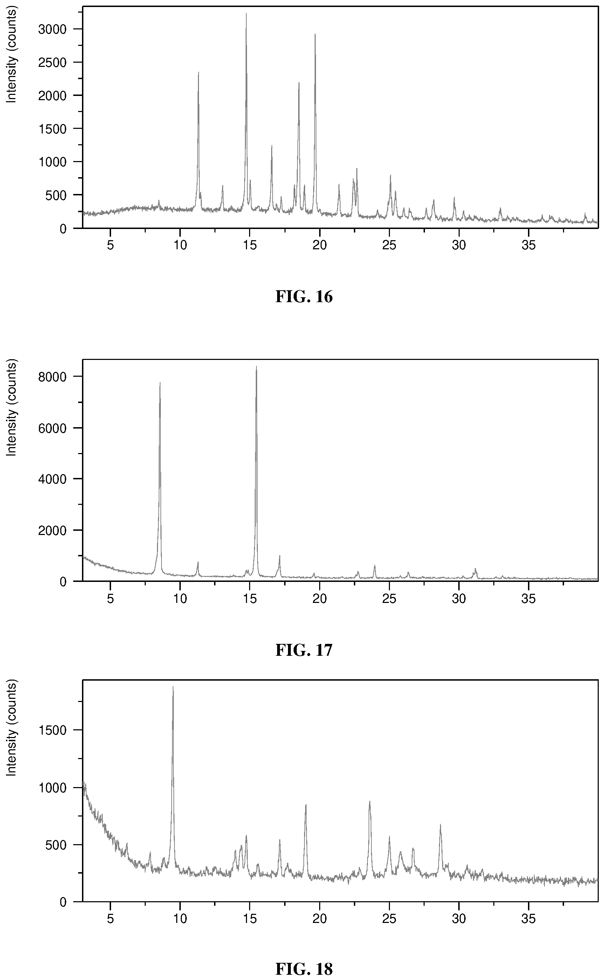

The compound (3) as a solid was suspended with stirring in methyl tert-butyl ether for 48 h and filtered to afford the type-XI crystal. Its XRPD pattern was as shown in FIG. 16.

The type-XI crystal showed characteristic peaks at diffraction angles of 20 values = 8.42°, 11.29°, 13.02°, 14.72°, 15.01°, 16.55°, 17.23°, 18.49°, 18.90°, 19.67°, 21.37°, 22.42°, 22.67°, 24.14°, 25.08°, 25.44°, 26.02°, 26.44°, 27.63°, 28.14°, 29.67°, 30.32°, 32.95°, 35.96°, 36.59°, and 39.03° in an X-ray powder diffraction pattern obtained by using Cu-Kα radiation.

**Table 11. XRPD Data on Type-XI Crystal**

| Peak Pos. [°2θ] | Rel. Int. [%] |
|---|---|
| 8.42 | 2.71 |
| 11.29 | 69.01 |
| 13.02 | 12.26 |
| 14.72 | 100.00 |
| 15.01 | 15.81 |
| 16.55 | 33.47 |
| 17.23 | 8.14 |
| 18.49 | 64.94 |
| 18.90 | 15.41 |
| 19.67 | 89.95 |
| 21.37 | 17.08 |
| 22.42 | 19.58 |
| 22.67 | 24.34 |
| 24.14 | 3.09 |
| 25.08 | 20.61 |
| 25.44 | 13.66 |
| 26.02 | 5.29 |
| 26.44 | 4.10 |
| 27.63 | 5.32 |
| 28.14 | 8.51 |
| 29.67 | 9.51 |
| 30.32 | 4.48 |
| 32.95 | 6.76 |
| 35.96 | 2.97 |
| 36.59 | 2.28 |
| 39.03 | 3.97 |

The TGA/DSC test results showed that the weight loss of the type-XI crystal was 19.3% when heated to 120°C, and multiple thermal signals occurred at approximate 95°C to 210°C.

### Experiment 14

### Type-XII Crystal

The type-III crystal or type-XI crystal of compound (3) was suspended with stirring in purified water at room temperature for about 24 h, filtered, and left open and dried overnight at room temperature to obtain the type-XII crystal. Its XRPD pattern was as shown in FIG. 17.

The type-XII crystal showed characteristic peaks at diffraction angles of 2θ values = 8.52°, 11.25°, 14.78°, 15.45°, 17.10°, 19.58°, 22.74°, 23.94°, 26.36°, 30.28°, 31.17°, and 33.11° in an X-ray powder diffraction pattern obtained by using Cu-Kα radiation.

**Table 12. XRPD Data on Type-XII Crystal**

| Peak Pos. [°2θ] | Rel. Int. [%] |
|---|---|
| 8.52 | 80.60 |
| 11.25 | 6.48 |
| 14.78 | 2.11 |
| 15.45 | 100.00 |
| 17.10 | 8.59 |
| 19.58 | 2.16 |
| 22.74 | 2.54 |
| 23.94 | 6.26 |
| 26.36 | 2.69 |
| 30.28 | 0.89 |
| 31.17 | 4.74 |
| 33.11 | 0.99 |

The type-XII crystal was vacuum dried at room temperature for 10 min and transformed into an amorphous crystal. The samples left open and dried at room temperature were characterized by TGA/DSC. The results showed that the weight loss was 59.6% when the samples were heated to 120°C, and multiple thermal signals occurred within the range of 50°C to 240°C.

### Experiment 15

### Type-XIII Crystal

The type-III crystal or type-XII crystal was suspended with stirring in methanol at room temperature for about 24 h, and filtered to afford the type-XIII crystal. Its XRPD pattern was as shown in FIG. 18.

The type-XIII crystal showed characteristic peaks at diffraction angles of 2θ values = 6.15°, 7.81°, 9.46°, 13.92°, 14.39°, 14.72°, 15.56°, 17.13°, 17.71°, 18.99°, 23.59°, 25.01°, 25.79°, 26.70°, 28.65°, and 30.56° in an X-ray powder diffraction pattern obtained by using Cu-Kα radiation.

**Table 13. XRPD Data on Type-XIII Crystal**

| Peak Pos. [°2θ] | Rel. Int. [%] |
|---|---|
| 6.15 | 6.08 |
| 7.81 | 6.94 |
| 9.46 | 100.00 |
| 13.92 | 13.20 |
| 14.39 | 17.75 |
| 14.72 | 21.73 |
| 15.56 | 6.59 |
| 17.13 | 21.65 |
| 17.71 | 6.16 |
| 18.99 | 44.51 |
| 23.59 | 44.06 |
| 25.01 | 23.40 |
| 25.79 | 14.48 |
| 26.70 | 15.76 |
| 28.65 | 31.80 |
| 30.56 | 5.10 |

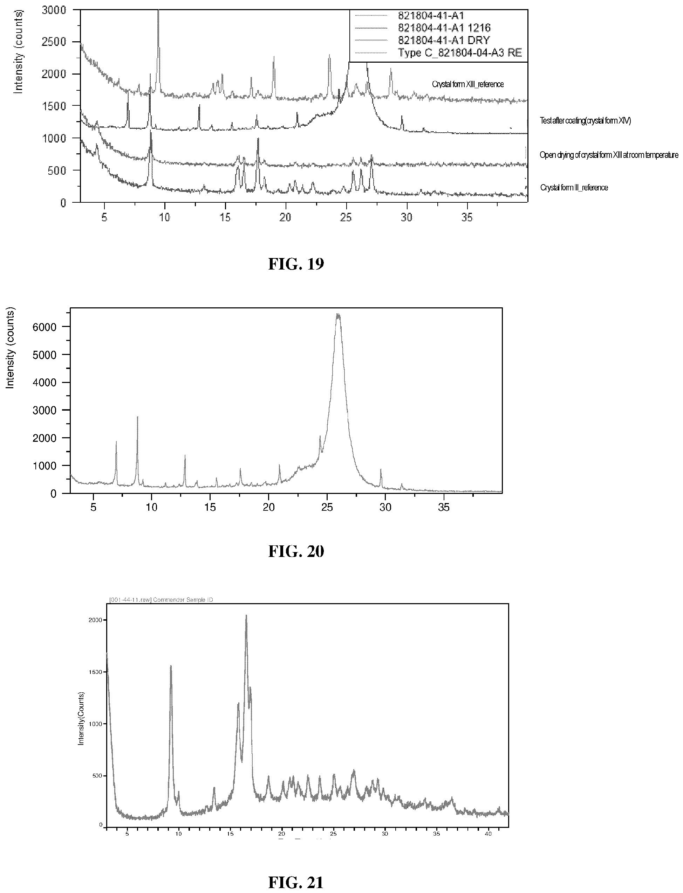

The type-XIII crystal was unstable, and the crystal form was transformed after left open and dried at room temperature for about 2 h. At the same time, the type-XIII crystal was coated to test XRPD, and a new type-XIV crystal could be observed. The type-XIV crystal was presumed to be in an unstable intermediate state, and would be transformed into the type-XIII crystal after removal from the solvent. The XRPD comparison diagram was as shown in FIG. 19. Rapid crystal form transformation of the type-XIII crystal was observed at room temperature.

### Experiment 16

### Type-XIV Crystal

The type-XIII crystal was left in a solvent environment at room temperature and transformed into the type-XIV crystal. Its XRPD pattern was as shown in FIG. 20. The type-XIV crystal was transformed into the type-XIII crystal after removal from the solvent environment.

The type-XIV crystal showed characteristic peaks at diffraction angles of 2θ values = 6.94°, 8.75°, 9.21°, 11.16°, 12.82°, 13.82°15.56°, 17.59°, 19.67°, 20.93°, 22.51°, 24.35°, 29.58°, and 31.37° in an X-ray powder diffraction pattern obtained by using Cu-Kα radiation.

**Table 14. XRPD Data on Type-XIV Crystal**

| Peak Pos. [°2θ] | Rel. Int. [%] |
|---|---|
| 6.94 | 76.15 |
| 8.75 | 100.00 |
| 9.21 | 10.76 |
| 11.16 | 6.46 |
| 12.82 | 49.49 |
| 13.82 | 900 |
| 15.56 | 14.81 |
| 17.59 | 27.13 |
| 19.67 | 4.02 |
| 20.93 | 36.18 |
| 22.51 | 25.25 |
| 24.35 | 62.81 |
| 29.58 | 36.07 |
| 31.37 | 11.21 |

### Experiment 17

### Type-XV Crystal

The type-XII crystal was left at 40°C to 90°C for 3 to 12 days to afford the type-XV crystal. Its XRPD pattern was as shown in FIG. 21.

The type-XV crystal showed characteristic peaks at diffraction angles of 2θ values = 9.247°, 9.982°, 13.4°, 15.781°, 16.55°, 16.929°, 18.704°, 20.14°, 20.78°, 21.1°, 22.497°, 23.654°, 25.013°, 25.667°, 27.026°, 28.186°, 22.715°, 28.822°, 29.265°, and 29.762° in an X-ray powder diffraction pattern obtained by using Cu-Kα radiation.

**Table 15. XRPD Data on Type-XV Crystal**

| Peak Pos. [°2θ] | Rel. Int. [%] |
|---|---|
| 9.247 | 81.6 |
| 9.982 | 8 |
| 13.4 | 12.1 |
| 15.791 | 53.7 |
| 16.55 | 100 |
| 16.929 | 62 |
| 18.704 | 13.2 |
| 20.14 | 8.2 |
| 20.78 | 9.9 |
| 21.1 | 10.5 |
| 21.54 | 7.8 |
| 22.497 | 12.8 |
| 23.654 | 12.6 |
| 25.013 | 13.2 |
| 25.667 | 6.3 |
| 27.026 | 14.9 |
| 28.186 | 5.4 |
| 28.822 | 9.9 |
| 29.265 | 10.2 |
| 29.762 | 6.1 |

The weight loss of the type-XV crystal was 2.2% when heated to 120°C, and the crystal form transformation occurred.

### Experiment 18

### Type-XVI Crystal and Type-XVII Crystal

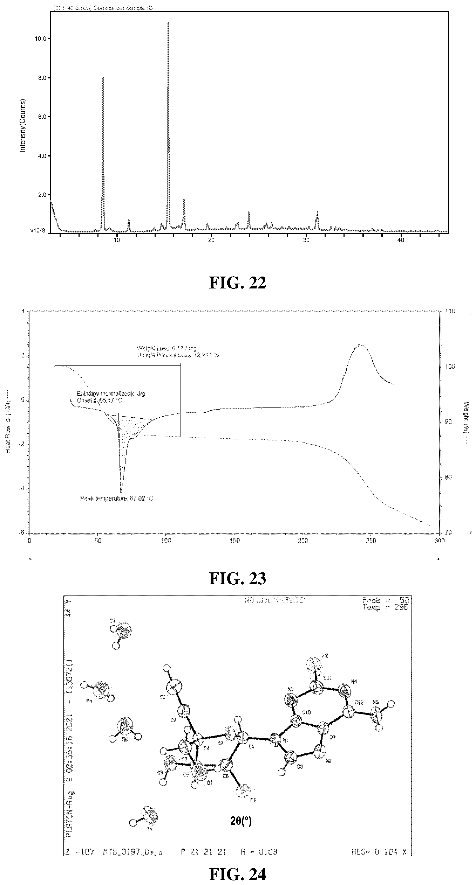

Compound (3) was suspended with stirring for 24 h in a mixed solvent of ethanol/water (volume ratio of 8:2) and filtered to afford the type-XVI crystal. The XRPD pattern was as shown in FIG. 22 and the DSC-TGA diagram was as shown in FIG. 23.

The single crystal data on the type-XVI crystal were as shown in Table 16.

**Table 16. Single Crystal Data on Type-XVI Crystal**

| | | |
|---|---|---|
| Labels | 001-40-1 (in solvent) | |
| Test formula | C12 H19 F2 N5 O7 | |
| Molecular weight | 383.32 | |
| Temperature | 296(2) K | |
| Wavelength | 1.54178 Å | |
| Crystal system | Orthorhombic | |
| Space group | P2₁2₁2₁ | |
| Cell parameters | a = 6.7413(7) Å | a= 90°. |
| | b = 11.9385(13) Å | b= 90°. |
| | c = 20.906(2) Å | g = 90°. |
| Volume | 1682.5(3) Å³ | |
| Z | 4 | |
| Density (calculated value) | 1.513 Mg/m³ | |

The three-dimensional molecular view of the single-crystal X-ray diffraction of the type-XVI crystal was as shown in FIG. 24, indicating that each type-XVI crystal contained four waters of crystallization.

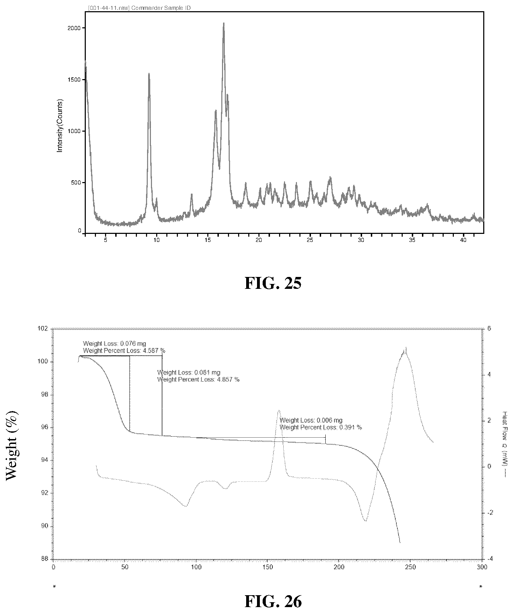

After vacuum drying at room temperature for 24 h, the type-XVI crystal showed an obvious dehydration phenomenon (weight loss: 14.39%), and turned into a monohydrate crystal form - type-XVII crystal after losing three waters of crystallization. The XRPD pattern was as shown in FIG. 25 and the DCS-TGA diagram was as shown in FIG. 26.

The type-XVI crystal was heated to 55°C in a nitrogen atmosphere and held for 2 to 24 h. The XRPD pattern showed that the type-XVII crystal was also obtained.

The type-XVII crystal was dried at 90°C for 1 to 24 h and lost one molecule of water (weight loss: 5.47%) to afford the type-XVIII crystal, which was an anhydride.

## Claims

1. Type-III crystal of compound (3), 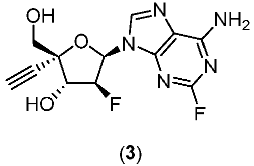 the type-III crystal showing characteristic peaks at diffraction angles of 2θ values = 8.83°, 16.08°, 16.53°, 17.67°, 18.19°, 25.52°, 26.20°, and 27.05° in an X-ray powder diffraction pattern obtained by using Cu-Kα radiation, wherein the 20 values are allowed to vary within an error range.

2. The type-III crystal of compound (3) according to claim 1, showing the diffraction peaks as shown in the following table:
| Peak Pos. [°2θ] | Rel. Int. [%] |
|---|---|
| 8.83 | 100.00 |
| 16.08 | 39.90 |
| 16.53 | 51.80 |
| 17.67 | 98.71 |
| 1.19 | 25.29 |
| 25.52 | 38.72 |
| 26.20 | 44.53 |
| 27.05 | 67.31 |

3. Method for preparing the type-III crystal of compound (3) according to claim 1 or 2, comprising: dissolving the compound (3) into a solution of 1,4-dioxane/DCM at a volume ratio of 1:10 to 5:10, adding petroleum ether or lower paraffin hydrocarbons to the solution, leaving or stirring for 1 to 24 h, preferably 3 to 10 h, more preferably 4 h to precipitate a crystal, and filtering the crystal; subjecting the resulting crystal to vacuum treatment at 25°C to 120°C, preferably 40°C to 60°C for 1 to 24 h, preferably 3 to 5 h to afford the type-III crystal.

4. Method for preparing the type-III crystal of compound (3) according to claim 1 or 2, comprising: stirring the compound (3) in ethanol at room temperature for 2 to 72 h, preferably 20 to 60 h, more preferably 48 h, filtering, and vacuum drying at room temperature to obtain a crystal, heating the resulting crystal to 120°C to 150°C, preferably 140°C to 145°C, more preferably 141°C under nitrogen protection to afford the type-III crystal of compound (3).

5. Method for preparing the type-III crystal of compound (3) according to claim 1 or 2, comprising: dissolving the compound (3) into THF or methyl THF, adding petroleum ether or lower paraffin hydrocarbons, which may include but not limited to pentane, hexane, octane, nonane, or decane or the like, to the resulting solution, and adding an appropriate amount of type III as a seed crystal to obtain a white suspension, stirring or leaving the resulting suspension at 40°C to 70°C, preferably 50°C for 1 to 4 h, preferably 60 to 70 min, and then slowly cooling to room temperature, and stirring or standing still for appropriate time; filtering the resulting suspension under reduced pressure at room temperature, washing with n-heptane, and subjecting to vacuum drying at 40°C to 80°C, preferably 50°C to afford the type-III crystal.

6. A pharmaceutical composition, comprising the type-III crystal of the compound (3) according to claim 1 or 2, and an optional pharmaceutical excipient.

7. Use of the type-III crystal of the compound (3) according to claim 1 or 2 or the pharmaceutical composition according to claim 6 in the preparation of a pharmaceutical or pharmaceutical formulation for resisting HIV or treating AIDS.
